# EUROPÄISCHE PATENTSCHRIFT

(11) **EP 4 387 571 B1**
(45) Veröffentlichungstag und Bekanntmachung des Hinweises auf die Patenterteilung: **02.07.2025**
(21) Anmeldenummer: 22762107.5
(22) Anmeldetag: 16.08.2022
(51) Int. Cl.: A61F 2/30

(54) **SCHEIBENFÖRMIGES AUGMENT FÜR EINEN KNOCHEN, INSBESONDERE RÖHRENKNOCHEN**
DISC-SHAPED AUGMENT FOR A TUBULAR BONE
INSERT EN FORME DE DISQUE POUR UN OS TUBULAIRE

(30) Priorität: 16.08.2021 EP 21000233; 08.08.2022 WO PCT/EP2022/072233
(43) Veröffentlichungstag der Anmeldung: 26.06.2024
(73) Patentinhaber: Waldemar Link GmbH & Co. KG, 22339 Hamburg (DE)
(72) Erfinder: LINK, Helmut D., 22397 Hamburg (DE)
(74) Vertreter: Glawe, Delfs, Moll
(86) Internationale Anmeldenummer: PCT/EP2022/072851
(87) Internationale Veröffentlichungsnummer: WO 2023/021030

(56) Entgegenhaltungen:
- EP-B1- 1 360 950
- DE-A1- 102006 047 663
- US-A1- 2012 209 391

## Beschreibung

Die Erfindung betrifft ein scheibenförmiges Augment zum Ausfüllen von Knochendefekten, insbesondere am Ende von langen Röhrenknochen wie Tibia. Das Augment ist versehen mit einer ersten Seite, einer zweiten Seite, einem Außenmantel an Lateralseiten sowie einer Innenwandung für eine von der ersten zur zweiten Seite laufende Durchgangsöffnung für einen Verankerungskiel einer an der zweiten Seite angeordneten Endoprothese.

Bei der Implantation von Endoprothesen, insbesondere Gelenkendoprothesen, tritt mitunter ein Problem auf, dass der die Endoprothese aufnehmende Knochen geschädigt ist, und zwar speziell im Bereich des Endes des Knochens (Knochenkopf). Grund hierfür sind insbesondere Defekte an der (spongiösen und/oder kortikalen) Knochensubstanz oder -oberfläche, bspw. aufgrund von Krankheit oder Verletzung, zunehmend aber auch aufgrund einer Explantation einer älteren Prothese. Um dennoch eine ausreichende Basis im Knochen zum Verankern der Endoprothese zu schaffen, werden typischerweise Augmente eingesetzt, die fehlende Knochensubstanz auffüllen. Es hat sich bewährt, sie versenkt im Knochenende anzuordnen, so dass sie von dem kortikalen Rand des Knochenendes köcherartig umgeben sind.

Scheibenartige Augmente zur Anwendung mit der Tibiakomponente einer Kniegelenkendoprothese sind bekannt beispielsweise aus der EP 1 360 950 B1. Vorgesehen sind unterhalb der Tibiaplatte zwei halbseitige Augmente, die links und rechts eines Verankerungskiels angeordnet sind. Die Augmente liegen bündig an der Unterseite der Tibiaplatte an und sind im montierten Zustand dort mittels einer Befestigungsschraube gesichert. Es hat sich gezeigt, dass auf diese Weise die Augmente zwar definiert in Bezug auf die Endoprothese gehaltert sind, jedoch nicht in Bezug auf den Knochenkopf, den sie eigentlich stützen bzw. dessen Knochendefekt sie an sich ausfüllen sollen. Ein stärkerer Bezug auf den Knochendefekt bzw. an den auszufüllenden Raum des Knochendefektes wäre wünschenswert. Eine weitere Schwierigkeit kann nach längerem Einsatz dadurch entstehen, dass im Laufe der Zeit das Augment im Knochen einwächst und danach die Endoprothese mit dem Augment, beispielsweise im Rahmen einer Revisionsoperation, nur schwer oder kaum zu entfernen ist. Es besteht dabei die Gefahr einer weiteren Schädigung des Knochens am ohnehin empfindlichen Knochenkopf.

Der Erfindung liegt die Aufgabe zugrunde, ein verbessertes Augment zu schaffen, mit dem die vorgenannten Nachteile vermieden oder verringert werden können.

Die erfindungsgemäße Lösung liegt in den Merkmalen des unabhängigen Anspruchs. Vorteilhafte Weiterbildungen sind Gegenstand der abhängigen Ansprüche.

Bei einem scheibenförmigen Augment zum Ausfüllen von Knochendefekten, insbesondere am Ende von langen Röhrenknochen wie Tibia, mit einer ersten Seite, einer zweiten Seite, einem Außenmantel an Lateralseiten sowie einer Innenwandung für eine von der ersten zur zweiten Seite laufende Durchgangsöffnung für einen Verankerungskiel einer an der zweiten Seite angeordneten Endoprothese, wobei das Augment eine generell C-förmige Gestalt mit zwei die Durchgangsöffnung flankierenden Schenkeln aufweist, ist erfindungsgemäß vorgesehen, dass ein Verbindungsstück zwischen den Schenkeln gelenkartig ausgeführt ist und mit den Schenkeln elastisch zusammenwirkt, so dass ein Rahmen gebildet ist, und bei Kompression der Schenkel eine nach außen gerichtete Rückstellkraft erzeugt ist, wobei die Schenkel ausgeführt sind in Skelettbauweise mit mehreren durch Schlitze getrennten, nebeneinanderliegenden Scheibensegmenten, die über Stege an dem Rahmen angeordnet sind.

Durch die elastische Ausführung des scheibenförmigen Augments wird ermöglicht, dass dieses am Knochenkopf in einer von der Kortikalis umgebenen Aushöhlung eingesetzt und versenkt werden kann. Hierbei kommt das scheibenförmige Augment mit seiner Außenfläche zur Anlage an die Innenseite der Kortikalis, und durch die beim Einsetzen vorgenommene Kompression der Schenkel wird die, vorteilhafterweise poröse, Struktur am Außenmantel gegen die Innenseite der Kortikalis gepresst. Dank der so erreichten Vorspannkraftmit wird eine innige Verbindung zwischen dem Außenmantel einerseits und der Kortikalis andererseits geschaffen.

Wegen der Skelettbauweise ist das scheibenförmige Augment nicht als eine unitäre Scheibe ausgeführt, sondern ist in mehrere nebeneinanderliegende Scheibensegmente unterteilt. Sie weisen typischerweise dieselbe Dicke auf. Die Scheibensegmente sind über Stege an dem Rahmen befestigt, der elastisch ist infolge des elastisch mit den Schenkeln zusammenwirkenden Verbindungsstücks. Damit ist eine Kompression der Schenkel ermöglicht, ohne dass es zu Verspannungen zwischen den Scheibensegmenten kommt. Die Skelettbauweise ermöglicht ein besonders günstiges Kompressions- und Elastizitätsverhalten, was das Einsetzen des Augments in einen am Knochenkopf geschaffenen Rezess vereinfacht und dort für eine erhöhte Befestigungssicherheit sorgt. Überrascherweise ist gewünschtenfalls auch eine ausreichende Dichtwirkung gegenüber Knochenzement gegeben, so dass eine durchgehende Dichtplatte nicht erforderlich ist. Ein weiterer Vorteil der Skelettbauweise ist, dass Augmente in verschiedenen Größenstufen leicht durch Hinzufügen/Vergrößern oder Weglassen/Verkleinern von einem oder mehreren Scheibensegmenten gebildet werden können, wobei typischerweise eine Vielzahl, also drei oder mehr Scheibensegmente vorgesehen sind. Die Skelettbauweise mit ihren zahlreichen Zwischenräumen, zwischen den Scheibensegmenten sowie zum elastischen Rahmen hin, ermöglicht aber nicht nur eine hohe Kompression, sondern begünstigt auch das Einwuchsverhalten von Knochenmaterial für eine langzeitstabile Befestigung.

Somit vereinigt die Erfindung gleich zwei wesentliche Vorteile in sich. Zum einen wird eine günstige Primärfixierung erreicht, und zum anderen werden beste Voraussetzungen für das nachfolgende Einwachsen von Knochenmaterial und damit für eine entsprechend solide langfristig stabile Befestigung geschaffen. Außerdem kann bei Bedarf zur Explantation das Augment mit seinem Außenmantel durch Kompression von der umgebenden Knochenstruktur gelöst werden, wodurch die Explantation erleichtert wird.

Nachfolgend seien einige verwendete Begriffe erläutert:
Unter der Kompression der Schenkel wird eine Bewegung der Schenkel aufeinander zu verstanden, wobei ein zwischen ihnen befindlicher Freiraum verengt wird. Das wird nachfolgend auch als Einfedern bezeichnet. Entsprechend ist eine entgegengerichtete Bewegung eine Expansion.

Unter einem Verankerungskiel einer Endoprothese wird eine Struktur verstanden, die zur Verankerung der Endoprothese in einem Knochen, insbesondere einem Markraum des Knochens vorgesehen ist und einen Schaft sowie seitlich daran angeordnete flügelartige Ausleger umfasst.

Die erste Seite des Augments ist typischerweise diejenige, die nach innen, also zum Knocheninneren hin orientiert ist. Die zweite Seite des Augments ist typischerweise diejenige, die in Gegenrichtung nach außen orientiert ist, also zum Knochenende hin.

Der Abstand zwischen der ersten und zweiten Seite der Scheibensegmente bestimmt die Dicke des Augments. Der Rahmen kann eine geringere Dicke aufweisen, ist aber vorzugsweise nicht dicker als die Scheibensegmente.

Unter dem "untermaßig" wird bei konturähnlichen Objekten verstanden, dass die Außenkontur des einen Objekts innerhalb der Außenkontur des anderen Objekts verläuft, und zwar vorzugsweise in den Abmessungen um ein bestimmtes absolutes oder relatives Maß verringert.

Unter einer geriffelten Struktur wird vorzugsweise eine grob geriffelte Struktur verstanden, mit einem Abstand zwischen einzelnen Profilen der Riffelung von mindestens einem Millimeter. Es hat sich gezeigt, dass eine solche grobe Riffelung mit Knochenzement eine zuverlässige und langzeitstabile formschlüssige Befestigung schafft.

Unter scharfkantig wird bei der geriffelten Struktur verstanden, dass vorstehende Bereiche der Riffelung ohne verrundete Kanten ausgeführt sind.

Die poröse Struktur weist vorzugsweise eine Porosität im Bereich von 60% bis 90% und/oder eine durchschnittliche Porengröße von 0,1 mm bis 1,5 mm auf, insbesondere von 0,4 mm bis 1,0 mm. Die Dicke der porösen Struktur beträgt vorzugsweise zwischen 0,6 mm und 2,5 mm, wobei die Dicke so bemessen ist, dass mindestens eine in der Tiefe des Materials (also nicht an der Oberfläche liegend bzw. angeschnitten) befindliche Schicht von Poren vorgesehen ist. Dadurch können in den Poren hinterschnittene Strukturen gebildet sein.

Vorzugsweise ist die erste Seite des scheibenförmigen Augments als poröse Struktur ausgeführt. Damit werden an der großen Kontaktfläche, mit der die erste Seite auf dem Knochen flächig anliegt, günstige Bedingungen für Knocheneinwuchs und somit gute Befestigung des Augments geschaffen. Mit Vorteil sind die Kanten massiv ausgeführt. Unter Kanten werden die Übergänge zwischen Seitenflächen verstanden, insbesondere von der ersten Seite zu lateralen Seiten. Damit wird eine Verstärkung der Kanten erreicht, die ein Ausbrechen der porösen Struktur verhindern.

Zweckmäßigerweise ist der Außenmantel als eine poröse, Knocheneinwuchs fördernde Struktur ausgeführt. Das begünstigt einen Knocheneinwuchs. Damit kann die durch die Kompression bewirkte Primärfixierung unterstützt werden durch eine langfristige Fixierung durch das eingewachsene Knochenmaterial.

Mit Vorteil ist die poröse Struktur so ausgeführt, dass sie in der Tiefe des Materials verbundene Poren umfasst. Die Porosität geht somit über eine oberflächliche Porosität hinaus und reicht bis in die Tiefe des Materials. Es werden so Hohlräume geschaffen, die miteinander in Verbindung stehen, wodurch besonders günstige Bedingungen für den Einwuchs von Knochenmaterial geschaffen sind. Mit Vorteil sind die Poren so dimensioniert, dass sie eine Weite von etwa 0,4 mm bis 1,0 mm aufweisende. Damit ergibt sich ein besonders günstiges Einwuchsverhalten. Zweckmäßig ist, wenn die poröse Struktur in der Tiefe mindestens eine und bis zu drei Schichten von Poren umfasst, was typischerweise eine bevorzugte Dicke der porösen Struktur von etwa 0,6 mm bis 2,5 mm bedeutet. Es ergeben sich so hinterschnittene Strukturen in den Poren, die bei eingewachsenem Knochenmaterial für eine gute Haltewirkung sorgen.

Zweckmäßigerweise ist vorgesehen, dass die poröse Struktur poröse Bereiche aufweist, die von einem massiven Rand umrahmt sind. Damit wird zum einen eine eindeutige Begrenzung erreicht und zum anderen ein unerwünschtes Ausbrechen von mechanisch empfindlicherem porösem Material aus der porösen Struktur, insbesondere im Radbereich, vermieden.

Es ist möglich, dass die Schenkel, insbesondere mit ihren Scheibensegmenten, aus einer porösen Struktur gebildet sind, wobei die zweite Seite zweckmäßigerweise mit einer Deckplatte versehen sein kann. Dies ergibt zum einen eine leichte Struktur, die zum anderen nahezu durchgängig offen für den Einwuchs von Knochenmaterial ist. Bevorzugt ist allerdings, dass die Schenkel einen massiven (nicht porösen) Kern aufweisen, an dessen Außenseiten Taschen gebildet sind, in denen die poröse Struktur angeordnet ist. Damit können definierte Zonen geschaffen werden, an denen die poröse Struktur vorhanden ist und wo ein Einwuchs des Knochenmaterials erfolgen solle. Außerdem werden so die Schenkel bzw. das Augment mechanisch robuster und belastbarer. Weitere Vorteil von Taschen mit begrenzter Tiefe in Bezug auf die Porosität sind die einfachere Herstellung sowie leichtere Reinigung.

Mit Vorteil ist die Innenwandung an dem Rahmen und/oder den Scheibensegmenten massiv ausgeführt. Damit kann ein unerwünschtes Einlaufen von Knochenzement in poröse Struktur des Rahmens bzw. der Scheibensegmente verhindert werden. Das gilt auch in Bezug auf andere laterale Flächen als den Außenmantel, bspw. gilt dies auch für Innenwände, zwischen denen Schlitze oder Spalte gebildet sind.

Vorzugsweise sind die Schlitze so eng bemessen, dass sie als Spaltdichtung für Knochenzement fungieren. Somit wird auf einfache und wenig aufwändige Weise eine ausreichende Abdichtung gegenüber Knochenzement erreicht. Vorzugsweise sind die Schlitze maximal 0,7 mm weit, weiter vorzugsweise maximal 0,4 mm. Je nach Höhe der Scheibensegmente und/oder Viskosität des Knochenzements können größere Weiten genügen oder es können kleinere Weiten erforderlich sein, um einen ausreichenden Dichtspalt zu erhalten.

Mit Vorteil sind die lateralen Flächen der Scheibensegmente (in der Regel sind dies deren laterale Außenseiten bzw. die Schlitze bildenden Seitenflächen) massiv ausgeführt, also nicht porös. Damit wird eine Aussteifung erzielt. Weiter wird so der Gefahr eines unerwünschten Eindringens von Knochenzement in bzw. durch die Schlitze wirksam begegnet. Bei einer solchen massiven Ausführung der lateralen Flächen braucht es auf die Weite der Schlitze nicht mehr anzukommen. Vorzugsweise sind die Scheibensegmente massiv ausgeführt, ggf. mit Taschen für poröse Struktur am Außenmantel und/oder an der ersten Seite.

Zweckmäßig ist, wenn der Rahmen ausgebildet ist als eine gekrümmte Blattfeder. Die durch die Kompression sich ergebende Spannungsbelastung verteilt sich auf den elastischen Rahmen, was zu einer gleichmäßigen Belastung und damit zu einer höheren Beanspruchungsfähigkeit führt. Besonders zweckmäßig ist es, wenn der Rahmen als ein die Gesamtheit der Scheibensegmente mindestens hälftig umschließender Außenrand ausgeführt ist, an dessen Außenseite der Außenmantel angeordnet ist. Der Rahmen berandet somit die Gesamtheit der Scheibensegmente, welche sich auf der Innenseite des Rahmens befinden. Der Rahmen kann so zugleich den Außenmantel mit der porösen Struktur bilden.

Mit Vorteil sind die Stege flexibel ausgeführt. Damit besteht eine Möglichkeit, dass sich die Scheibensegmente relativ zu dem Rahmen bewegen. Es ist so eine laterale Ausweichbewegung des jeweiligen Scheibensegments eröffnet, was insbesondere bei starker Kompression von Vorteil sein kann.

Vorzugsweise ist in dem Rahmen und/oder den Scheibensegmenten mindestens ein Befestigungsloch zur Aufnahme einer Befestigungsschraube vorgesehen. Damit kann eine zusätzliche Befestigung des Augments am Knochen erreicht werden, was insbesondere einen wertvollen Beitrag zur Primärbefestigung leisten kann. Mit Vorteil weist das Befestigungsloch einen massiven Lochmantel auf. Durch einen solchen massiven Lochmantel wird eine Aussteifung erreicht, um die Stabilität des Befestigungslochs auch unter Einwirkung der Anzugkraft der Befestigungsschraube zu gewährleisten. Ferner kann so ein unerwünschtes Einwachsen von Knochenmaterial verhindert werden.

Für die Skelettbauweise ist es zweckmäßig, wenn das Befestigungsloch in einem der Scheibensegmente angeordnet ist. Vorzugsweise ist dieses Scheibensegment nicht mittels eines schmalen Stegs am elastischen Rahmen angeordnet ist, sondern mittels einer breiteren Brücke, die weiter vorzugsweise mindestens um eine halbe Stegbreite breiter ist (also mindestens die 1,5-fache Breite eines Stegs aufweist). Damit werden zwei Vorteile erreicht: zum einen wird eine leistungsfähige Befestigung des durch die Befestigungsschraube zusätzlich belasteten Scheibensegments bewirkt. Zum anderen ermöglicht es die breitere Brücke, das Befestigungsloch näher an den Rand und damit in die Nähe des Rahmens zu rücken, was insbesondere bei kleinen Größen des Augments durch die so verbesserte Raumausnutzung ein erheblicher Vorteil ist. Es können auch mehrere Scheibensegmente mit einem Befestigungsloch versehen sein.

Die Durchgangsöffnung ist typischerweise ein langgestreckter Aufnahmeraum für einen Verankerungskiel der Endoprothese. Die Durchgangsöffnung kann zweckmäßigerweise zu einer Seite hin offen sein. Sie liegt dann am Rand des Augments als Randöffnung. Dies ist besonders dann ein Vorteil, wenn das Augment als halbseitiges Augment ausgeführt ist. Der Begriff halbseitig bezieht sich hierbei auf die Abmessungen der Endoprothese, deren Verankerungskiel durch die Durchgangsöffnung des Augments geführt ist. Die Endoprothese benötigt häufig nur auf einer Seite Unterfütterung, beispielsweise wenn ein Knochendefekt auf eine Seite des Knochenkopfs beschränkt ist und das Augment daher nur dort benötigt wird. Es versteht sich, dass auch zwei halbseitige Augmente vorgesehen sein können, um beide Seiten zu unterfüttern.

Die Durchgangsöffnung ist typischerweise eher klein bezogen auf die Abmessungen des Augments, in der Regel nimmt sie weniger als die Hälfte, häufig weniger als ein Drittel des Volumens des Augments ein. Dies hat den Vorteil, dass somit noch ausreichend stützendes Material für die Schenkel bzw. die Scheibensegmente verbleibt, um eine ausreichende Unterfütterung zu gewährleisten. Die Durchgangsöffnung ist in der Regel so bemessen, dass sie mit geringem Spiel den Verankerungskiel ggf. dessen flügelartige Ausleger aufnimmt. Das geringe Spiel ist zweckmäßigerweise so groß bemessen, dass eine gewünschte Kompression, die durch das elastische Einfedern des Rahmens zu einer Verkleinerung der Durchgangsöffnung führt, ermöglicht ist, ohne an den Verankerungskiel anzuschlagen. Die Erfindung kann sich auch auf solche Augmente erstrecken, die zwar eine Durchgangsöffnung haben, aber nicht für einen Verankerungskiel.

Der Außenmantel steht mit Vorteil nicht in einem 90° Winkel zur ersten Seite bzw. zur zweiten Seite. Es hat sich bewährt, wenn der Außenmantel konisch geneigt ist, vorzugsweise mit einem Winkel von 5 bis 10° zur ersten Seite hin verjüngend. Damit wird eine Anpassung an eine sich verjüngende Außenkontur des Knochens erreicht, und zum anderen kann dank dieser Konizität kann das Augment leichter durch Zusammendrücken eingesetzt bzw. entnommen werden, als dies bei senkrecht stehendem Außenmantel der Fall wäre.

Zweckmäßigerweise sind an dem Außenmantel nach außen gerichtete Spikes angeordnet. Sie pressen sich im implantierten Zustand in die umlaufende Kortikalis des Knochenkopfes, in welchem das Augment in einem Rezess aufgenommen ist. Damit wird einer Erhöhung der Befestigungssicherheit erreicht, und zwar bereits von Beginn an, also eine verbesserte Primärfixierung. Beim Zusammendrücken (Kompression) der Schenkel des Augments zur Explantation bewegen sich die Spikes dann selbsttätig zurück aus der Kortikalis, wodurch sie die Explantation freigeben.

An der zweiten Seite kann eine Deckplatte vorgesehen sein, die eine geriffelte Struktur als Verzahnung für eine Zementverbindung aufweist. Damit kann eine zusätzliche Befestigung erreicht werden und es wird eine Durchschneideschicht geschaffen, welche im Fall einer geplanten Explantation auf verhältnismäßig einfache Weise mittels einer Säge zu durchtrennen ist, wodurch die Endoprothese freikommt von dem Augment und somit leicht entnommen werden kann, ohne dass ggf. stark in den Knochenkopf eingewachsene Augment herauszureißen. Mit Vorteil ist die geriffelte Struktur genutet ausgeführt. Zweckmäßigerweise sind durchlaufende Nuten vorgesehen, die vorzugsweise in eine Richtung im Wesentlichen quer zur Erstreckung der Durchgangsöffnung orientiert sind. Durch die Nutung wird eine besonders wirksame Verzahnung mit einem aufgebrachten Zementbett erreicht. Die Nutung weist vorzugsweise solche Abstände und Höhe auf, die auf das Fließ- und Abbindeverhalten sowie die Körnung des Zementbetts abgestimmt ist. Bewährt haben sich Nut-Abstände im Bereich von 2 bis 10 mm und/oder einer Nut-Tiefe von 0,5 bis 2 mm, wobei die Nuten begrenzende Profile etwa 0,5 bis 3 mm breit sind.

Die geriffelte Struktur kann scharfkantig ausgeführt sein. Jedoch sind auch andere alternative Profilformen denkbar, hilfsweise dreieckige oder Profilformen mit Verrundungen an den Ecken.

Zweckmäßig ist, wenn die geriffelte Struktur durch ein Drahterodier-Verfahren hergestellt ist. Damit können auf effiziente Weise nahezu beliebige Profilformen, darunter auch scharfkantige, effizient gebildet sein.

Besonders zweckmäßig ist es, wenn das Augment einheitlich durch ein additives Verfahren, insbesondere 3D-Druck, hergestellt ist. Damit können auch komplexe Formen in verschiedenen Größen ohne oder lediglich mit einem Minimum an aufwendiger Nachbearbeitung erzeugt werden. Besonders zweckmäßig ist eine Kombination mit dem Drahterodieren zum Schaffen definierter, scharf begrenzter Profile, wie vorstehend erwähnt.

Es kann eine zementdichte Deckplatte vorgesehen sein. Unter zementdicht wird verstanden, dass kein Knochenzement durch die Deckplatte tritt. Dazu kann die Deckplatte durchgehend mit schlitzartigen Durchbrechungen ausgeführt sein. Es wird so oben auf das Augment aufgebrachter Knochenzement separiert von dem inneren und unteren Bereich des Augments, sodass dort kein Knochenzement einfließt und den Einwuchs von Knochenmaterial stört. Die Deckplatte hindert nicht das elastische Verhalten des Rahmens mitsamt der Kompression und die relative Beweglichkeit der Scheibensegmente.

Mit Vorteil ist vorgesehen, dass die poröse Struktur mit einer knocheneinwuchsfördernden Beschichtung, insbesondere umfassend Kalziumphosphat, versehen ist, und/oder an nicht-porösen Bereichen mit einer bioziden Beschichtung, insbesondere umfassend Silber, versehen ist. Damit wird erreicht, dass an gewünschten Stellen das Einwachsen von Knochenmaterial begünstigt wird und an anderen Stellen, an denen dies insbesondere zum Zweck einer einfacheren Explantation nicht gewünscht ist, dies nicht geschieht.

Vorzugsweise ist an dem Rahmen mindestens ein Biegescharnier vorgesehen, das dazu ausgebildet ist, eine zusätzliche Kompression der Schenkel zu ermöglichen und eine nach außen gerichtete Rückstellkraft zu erzeugen. Damit wird - zusätzlich zu dem blattfederartigen Einfedern des Rahmens als Ganzes - ein lokalisiertes Biegescharnier geschaffen, wodurch eine stärkere Kompression erreicht wird, die insbesondere schon bei geringerer Kraft wirksam wird. So kann eine Veränderung der Federhärte des elastischen Rahmens erreicht werden. Insbesondere für kleine Größen der Augmente ist das ein Vorteil, da diese ansonsten wegen ihrer Kompaktheit mitunter zu steif wären. Der Begriff "lokalisiert" wird vorliegend im Sinne von räumlich konzentriert verstanden. Mit Vorteil können mehrere solcher lokalisierter Biegescharniere vorgesehen sein, um eine gleichmäßigere Verteilung der zusätzlichen Kompression und der Kraftbeaufschlagung durch die Rückstellkraft zu erreichen. Mehrere Biegescharniere begünstigen auch eine weitgehend formtreue Kompression.

Es ist zweckmäßig, dass das mindestens eine Biegescharnier gebildet ist durch eine lokale Materialschwächung, insbesondere in Form einer Nut, einer Schlitzung und/oder Perforationen. Damit können auf fertigungstechnisch günstige Weise Biegescharniere am Rahmen geschaffen werden. Die Ausführung als Nut bietet den Vorteil, dass der Nutgrund die durch den Rahmen bewirkte Separierung von Innen- und Außenraum bewahrt. Eine Schlitzung bietet den Vorteil, dass damit eine kleinere Biegesteifigkeit eingestellt werden kann, wobei bei entsprechend schmaler Gestaltung der Schlitze (für bevorzugte Weiten s.o.) ein Schutz vor unerwünschtem Zementdurchfluss besteht. Die Ausführung als insbesondere runde Perforationen bietet den Vorteil, dass sie rationell herstellbar ist und weiter den Vorteil besitzt, ggf. durch Bohren weiterer Löcher auch eine nachträgliche Anpassung zu ermöglichen. Bevorzugt sind daher runde, insbesondere kreisrunde Perforationen. Sie bieten ferner den Vorteil, dass sie mangels Ecken einer Lastkonzentration entgegenwirken und daher eine günstige Lastverteilung auf den umgebenden Bereich des Biegescharniers ermöglichen. Mehrere Schlitzungen und insbesondere Perforationen können auch in Gruppen zusammengefasst sein. Es können so auch geringe Rückstellkräfte eingestellt werden, was insbesondere bei kleinen Größen von Augmenten einen beträchtlichen Vorteil darstellt. - Anpassungen können ferner erfolgen durch Materialwahl, so ist eine Titanlegierung wie Ti6Al4V steifer als Reintitan (z.B. Titan Grade 2). Variationen der Breite oder Tiefe der Nut bzw. die Länge und Breite der verbleibenden Stege zwischen den Schlitzen/Perforationen ermöglichen ebenfalls Anpassungen in Bezug auf die Biegesteifigkeit der Biegescharniere. Mit Vorteil können bei mehreren Biegescharnieren diese sich bezüglich ihrer Materialschwächung unterscheiden, so dass sie bei Kompression verschieden hohe Rückstellkräfte aufweisen. Dies ermöglicht eine feinere Einstellung der Biegesteifigkeit, und zwar bezüglich Härte und Ort.

Die Erfindung erstreckt sich ferner auf eine Anordnung aus einer Endoprothese, insbesondere einer Kniegelenkendoprothese, und dem scheibenförmigen Augment. Ein Verankerungskiel der Endoprothese ist in die Durchgangsöffnung aufgenommen, und zwar insbesondere im implantierten Zustand. Zweckmäßigerweise ist das scheibenförmige Augment in seiner Außenkontur ähnlich der Endoprothese gestaltet, insbesondere einer Tibiaplatte. Jedoch ist es gegenüber dieser untermaßig, d. h. es weist etwas kleinere Abmessungen auf (insbesondere im Bereich von 2 bis 6 mm entlang des Außenmantels). So kann es die Endoprothese, insbesondere Tibiaplatte, unterstützen, aber dennoch in einem von der Kortikalis berandeten Rezess am Knochenkopf aufgenommen sein.

Mit Vorteil ist das scheibenförmige Augment zur Unterstützung einer linken oder rechten Hälfte der Endoprothese halbseitig ausgeführt. Damit kann eine wirksame Unterstützung durch das Augment erreicht werden, wenn ein Knochendefekt nur einseitig am Knochenkopf vorliegt, und so das unnötige Entfernen von Knochenmaterial an der anderen, gesunden Seite vermieden werden.

Zweckmäßigerweise ist die Durchgangsöffnung zur Aufnahme von dem Verankerungskiel und dessen flügelartigen Auslegern mit einem definierten Freiraum von vorzugsweise 1 bis 3 mm bemessen. Mit diesem Freiraum entsteht ein Spiel zwischen dem Verankerungskiel mit dessen flügelartigen Auslegern und den Schenkeln des Augments, wodurch ein Toleranzausgleich sowie das Einfedern des Rahmens durch Kompression ermöglicht sind. Für die Praxis ist es eine erhebliche Erleichterung, da so eine große Kompression sowie eine unerwünschte Verklemmung von Augment und Verankerungskiel mit seinen flügelartigen Auslegern vermieden werden kann.

Die Erfindung wird nachfolgend unter Bezugnahme auf die beigefügte Zeichnung anhand vorteilhafter Ausführungsbeispiele erläutert. Es zeigen:
- Fig. 1: eine perspektivische Ansicht eines ersten Ausführungsbeispiels für ein scheibenförmiges Augment;
- Fig. 2A, B: eine Rückansicht sowie eine Schnittansicht von medial des Augments gemäß Fig. 1;
- Fig. 3A-F: verschiedene Profilformen für eine Riffelung;
- Fig. 4: eine Frontalansicht des Augments zusammen mit einer Tibiaplatte einer Kniegelenkendoprothese;
- Fig. 5A, B: Aufsichten auf eine erste Seite des Augments in zwei verschiedenen Größen;
- Fig. 6: eine Explosionsansicht des Augments gemäß Fig. 1 mit der Kniegelenkendoprothese;
- Fig. 7: eine perspektivische Ansicht zur Anordnung des Augments an der Kniegelenkendoprothese;
- Fig. 8: eine Aufsicht auf eine erste Seite eines zweiten Ausführungsbeispiels für ein scheibenförmiges Augment mit Biegescharnieren; und
- Fig. 9A, B: Detailansichten von Varianten zur Einstellung einer Biegesteifigkeit der Biegescharniere.

Die Erfindung wird nachfolgend erläutert anhand eines Beispiels für ein Augment für eine Kniegelenkendoprothese, und zwar genauer gesagt für ein am oberen (proximalen) Knochenkopf der Tibia angeordnetes Augment. Das Augment ist zu unterscheiden von der Prothese, d.h. das Augment ist kein Element der eigentlichen Prothese. Das Augment verstärkt den Knochen und erhöht bzw. verbessert so dessen Aufnahmefähigkeit für die Prothese. Erfindungsgemäße Augmente können selbstverständlich auch am anderen (distalen) Ende oder auch an anderen Knochen vorgesehen sein. Das Augment 1 besteht vorzugsweise aus einer Titanlegierung (bspw. Ti6Al4V) oder Reintitan (bspw. Titanium Grade 2), und kann zweckmäßigerweise durch ein additives Verfahren (3D-Druck) hergestellt sein. Es kann aber auch aus anderem biokompatiblem Material bestehen, beispielsweise aus metallischem Material wie bspw. Kobalt-Chrom-Molybdän (CoCrMo), rostfreiem Stahl (Edelstahl) oder auch aus Kunststoffmaterial, wie bspw. Polyetheretherketon (PEEK).

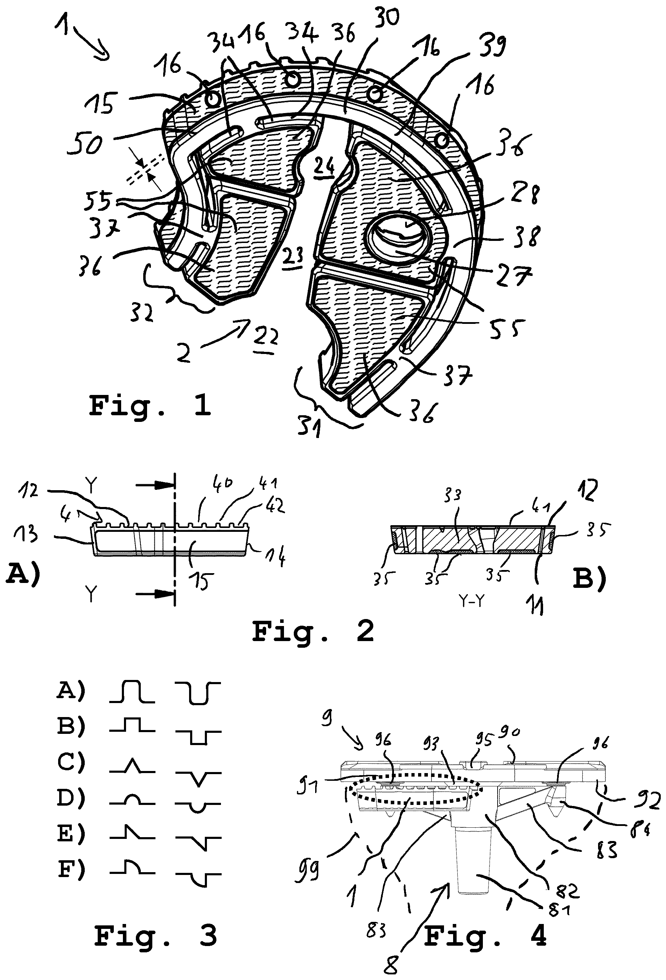

Das Augment 1 des vorliegend erläuterten Ausführungsbeispiels ist dazu vorgesehen, unterhalb der Tibiakomponente 9 einer Kniegelenkendoprothese angeordnet zu sein, wie sie in Figur 4 dargestellt ist. Die Kniegelenkendoprothese umfasst eine am Femurknochen (nicht dargestellt) anzuordnende femorale Komponente (nicht dargestellt) sowie die am proximalen Ende eines Tibiaknochens 99 anzuordnen Tibiakomponente 9. Diese umfasst eine Tibiaplatte 91, die lateral ausgedehnt auf einem resezierten Knochenkopf der Tibia angeordnet ist. Die erste (distale) Seite 92 der Tibiaplatte 91 ist ausgebildet zur Anlage an der Oberfläche des Knochenkopfs der Tibia 99. Auf der gegenüberliegenden zweiten (proximalen) Seite (Oberseite) der Tibiaplatte 91 ist eine Aufnahme 90 vorgesehen, in welche ein Lagerstück (nicht dargestellt) der Kniegelenkendoprothese anzuordnen ist. Je nachdem ob die Tibiakomponente zur zementierten oder zementfreien Implantation vorgesehen ist, ist die Tibiaplatte 91 an ihrer ersten (distalen) Seite 92 optional mit einer porösen Struktur zum Einwuchs von Knochenmaterial versehen, um so bei zementfreier Implantation eine optimale Befestigung zu ermöglichen.

Zur Befestigung der Tibiaplatte 91 ist ein Verankerungskiel 8 vorgesehen, welcher nach distal abragt. Er weist ein Schaftstück 82 und ein sich daran distal anschließendes Konusstück 81 auf. Das Konusstück 81 ist dazu ausgebildet, ggf. einen in den Markkanal der Tibia 9 ragenden Steckschaft aufzunehmen. Nach lateral schließen sich an das Schaftstück 82 jeweils ein flügelartiger Ausleger 83 sowohl zur linken wie zur rechten Seite an, an dessen freiem Ende eine Spannhülse 84 angeordnet ist. Die flügelartigen Ausleger 83 fungieren als Tragarme für die Tibiaplatte 91. Die Tibiaplatte 91 kann somit an drei Punkten mit dem Verankerungskiel 8 verbunden sein, nämlich zentral an dem Schaftstück 82 und jeweils links- und rechtsseitig mit den an freien Enden der flügelartigen Ausleger 83 angeordneten Spannhülsen 84. Dazu sind an der Tibiaplatte 91 zwei Verschraubungslöcher 94 links und rechts vorgesehen, die mit der jeweiligen Spannhülse 84 fluchten.

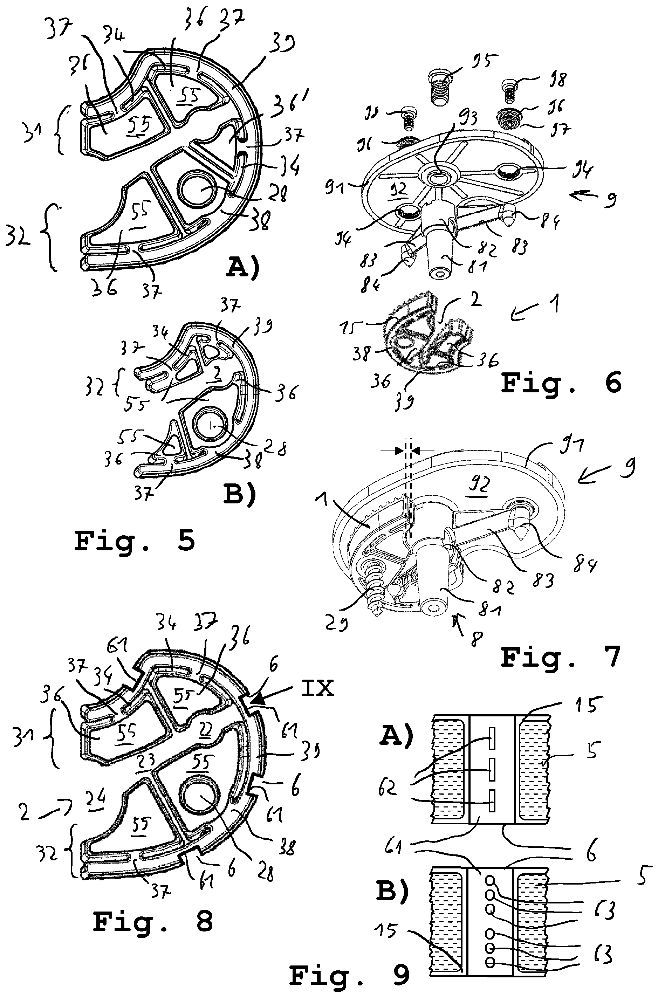

Wie Figur 4 zeigt, liegen hierbei die flügelartigen Ausleger 83 nicht bündig an der Unterseite 92 an, sondern sind mit einem definierten Abstand gehaltert. Dazu ist an der Unterseite 92 ein zentraler Auflagebund 93 vorgesehen, an welchem das Schaftstück 82 zur Anlage kommt. In die Verschraubungslöcher 94 sind Abstandseinsätze 96 mit einem Außengewinde 97 eingeschraubt (s. Fig. 6), welche einen definierten Abstand zu den Spannhülsen 84 herstellen. Auf diese Weise wird zusammen mit dem zentralen Auflagebund 93 ein definierter Abstand zwischen der Unterseite 92 der Tibiaplatte 91 und den flügelartigen Auslegern 83 des Verankerungskiels 8 eingestellt. Dies ist in dort durch die mit gepunkteter Linie dargestellte Ellipse hervorgehoben.

Eine Explosionsdarstellung ist in Figur 6 dargestellt. Man erkennt als Hauptkomponenten das Augment 1 und die Tibiaplatte 91 mit ihrem Verankerungskiel 8. Wie bereits vorstehend beschrieben, erfolgt eine Montage bzw. Zusammenbau, indem der Verankerungskiel 8 mit einem Schaftstück 82 mittels der Zentralschraube 95 unten an der distalen Seite 11 der Tibiaplatte 91 befestigt ist. Zusätzlich sind Abstandseinsätze 96 aus Titanmaterial mit einem Außengewinde 97 in die Verschraubungslöcher 94 eingeschraubt und bestimmen so einen definierten Abstand zwischen den flügelartigen Auslegern 83 und der distalen Seite 92 der Tibiaplatte 91. Zur zusätzlichen Befestigung und Abstützung sind weiter Befestigungsschrauben 98 vorgesehen, welche durch den jeweiligen Abstandseinsatz 96 durchgeführt sind und die in ein Innengewinde der jeweiligen Spannhülse 84 eingreifen und diese somit gegen den Abstandseinsatz 96 spannen. Auf diese Weise wird ein definierter Abstand zwischen den flügelartigen Auslegern 83 und der distalen Seite 92 der Tibiaplatte 91 eingestellt.

Figur 1 zeigt eine perspektivische Ansicht auf die erste (distale) Seite 11 eines ersten Ausführungsbeispiels für ein Augment 1 gemäß der vorliegenden Erfindung. Das Augment 1 ist wie eine dicke Scheibe geformt und weist zwei Schenkel 31, 32 auf, die über ein Verbindungsstück 30 am gemeinsamen Ende der Schenkel 31, 32 gelenkartig elastisch gekoppelt sind. Es ist so ein elastischer Rahmen 39 gebildet, der typischerweise etwa ein Dreiviertel des Umfangs des Augments 1 umfasst. Die Schenkel 31, 32 sind gegliedert in Scheibensegmente 36, wobei zwischen den Schenkeln 31, 32 eine Durchgangsöffnung 2 angeordnet ist, die zur Aufnahme eines Verankerungskiels 8 der Tibiakomponente 9 ausgebildet ist. Mit dem Rahmen 39 und den Scheibensegmenten 36 ist das Augment 1 in Skelettbauweise ausgeführt.

Die Scheibensegmente 36 sind jeweils über einen Steg 37 innen an dem Rahmen 39 angeordnet. Somit sind zwischen den Scheibensegmenten 36 sowie zwischen Scheibensegmenten 36 und der Innenseite der Wandung des Rahmens 39 schmale Schlitze 34 gebildet, die Freiraum für eine relative Bewegung der Scheibensegmente 36 bei einer Kompression/Expansion bieten. Die Weite der Schlitze 34 kann so bemessen sein, dass unter Berücksichtigung der durch die Dicke der Scheibensegmente 36 des Augments 1 vorgegebenen Tiefe der Schlitze 34 eine gegen den Eintritt von Knochenzement wirksame Spaltdichtung gebildet wird. Mit diesen Scheibensegmenten 36 kann eine feine Anpassung an den Raumbedarf der Durchgangsöffnung 2 erfolgen, welcher wiederum von der Beschaffenheit des Verankerungskiels 8 der zu implantierenden Prothese maßgeblich bestimmt ist.

Eine Rückansicht des Augments 1 sowie eine Schnittansicht von medial sind den Figuren 2A und B dargestellt. Die Rückansicht bezieht sich hierbei auf die Orientierung des Augments 1 im eingesetzten Zustand. Unter Nutzung anatomischer Fachausdrücke handelt es sich also um eine Ansicht von posterior. Oben, also im Ausführungsbeispiel auf der zweiten (proximalen) Seite, auf dem Augment 1 kann eine zementdichte Deckplatte 4 ausgebildet sein. Diese hindert nicht das elastische Verhalten des Rahmens 39 mitsamt der Kompression und die relative Beweglichkeit der Scheibensegmente 36. Die Deckplatte kann auf ihrer Oberseite mit einer geriffelten Struktur 40 versehen sein. Die geriffelte Struktur 40 kann so als Verzahnung zu einem Zementbett (nicht dargestellt) dienen, welches den Zwischenraum zu der Tibiaplatte 91 auffüllt, und sorgt so für eine zusätzliche Befestigung. Die Riffelung 40 kann eine Nutung 42 aufweisen, wobei die einzelnen Nuten durch erhabene Profile voneinander abgegrenzt sind. Die Nutung kann in eine Richtung von vorne nach hinten orientiert sein, also bezogen auf den implantierten Zustand von anterior nach posterior. Die Tiefe der Nutung 40 kann bspw. 1 mm, die Breite der Nutung etwa 3 bis 4 mm und die Breite der erhabenen Profile 41 beträgt etwa 1,5 mm. Im einfachsten Fall können die Profile 41 eine rechteckige Querschnittsform aufweisen, wie sie in Figur 3A) oder 3B) dargestellt sind, mit verrundeten bzw. scharfen Ecken, jeweils als positives oder negatives Profil. Alternativ sind auch nicht rechteckige Formen für das Profil möglich, beispielsweise in Form eines symmetrischen Dreiecks oder eines asymmetrischen, sägezahn-artigen Dreiecks, wie in Figur 3C) bzw. 3E) dargestellt. Ferner können auch verrundete Profile vorgesehen sein, beispielsweise nach Art eines Halbrunds wie in Figur 3D) oder asymmetrisch nach Art eines Viertelkreises wie in Figur 3F) dargestellt. Dies kann der Fachmann je nach Anforderungen an die durch die Profile 41 gebildete Verzahnung mit dem Knochenzement auswählen.

In Figur 2A, B erkennt man ferner an den jeweiligen lateralen Seiten des Augments 1, dass diese konisch zum Knocheninneren zur ersten Seite hin (vorliegend nach distal, in der Abbildung nach unten) zulaufend sind. In dem dargestellten Ausführungsbeispiel beträgt der Winkel auf jeder Seite etwa 7°. Es können auch andere Winkel vorgesehen sein. Die konische Gestaltung vereinfacht nicht nur das Einsetzen bei der Implantation, sondern vor allem auch ein Entnehmen des Augments 1 aus dem Knochen im Rahmen einer Explantation. Man erkennt ferner, dass der Rahmen 1 und die Scheibensegmente 36 etwa die gleiche Dicke aufweisen.

Es wird nun Bezug genommen auf die Figuren 1 und 2B). Wie in Figur 1 durch strichlierte Darstellung visualisiert ist, sind die erste (distale) Unterseite 11 des Augments 1 sowie ein Außenmantel 15 an der Lateralseite 13 jeweils mit einer porösen Struktur 5 versehen. Bei der dargestellten Ausführungsform geht die poröse Struktur nicht durch, sondern die Schenkel 31, 32 weisen einen massiven Kern 33 auf. Es sind Taschen 35 an den entsprechenden Oberflächen der Schenkel 31, 32 vorgesehen, in denen die poröse Struktur 5 angeordnet ist. Die Taschen 35 weisen eine Tiefe von etwa 0,8 bis 2 mm auf. In Figur 2B sind derartige Taschen an der ersten (distalen) Seite 11 sowie lateral an dem Außenmantel 15 vorgesehen und dargestellt. Um ein unerwünschtes Ausbrechen der porösen Struktur 5 insbesondere an den Rändern des Augments 1 zu verhindern, sind die Kanten zwischen benachbarten porösen Strukturen 5 am Übergang von der distalen Seite zur Lateralseite mit massiv, also nicht porös ausgeführten Kanten 50 versehen. Somit wird dort eine Verstärkung erreicht und ein Ausbrechen der porösen Struktur 5 verhindert. Die Breite dieser Kanten 50 ist durch die beiden gegenläufigen Pfeile in Figur 1 visualisiert und beträgt vorzugsweise etwa 1 mm bis 2 mm.

Ferner können an dem Außenmantel 15 radial nach außen weisende Spikes 16 angeordnet sein. Diese bohren sich nach der Implantation des Augments 1 in die umlaufende Kortikalis des Tibiakopfs und sichern somit das Augment 1 zusätzlich in seiner Position.

In eines der Scheibensegmente 36 kann das Befestigungsloch 28 angeordnet sein. Es ist so bemessen, dass als zusätzliche Befestigung eine Spongiosaschraube 29 durch diese Öffnung gesteckt und angezogen werden kann. Zur zusätzlichen Aussteifung ist das Befestigungsloch 28 mit einem massiven Lochmantel 27 als Innenauskleidung versehen. Eine solche Spongiosaschraube 29 zur zusätzlichen Befestigung ist in Figur 7 dargestellt.

Um das Befestigungsloch 28 näher an den Rand des Scheibensegments bringen zu können, und zwar vorzugsweise in die Nähe des umlaufenden Rahmens 39, ist zweckmäßigerweise der Steg an dieser Stelle deutlich verbreitert zu einer Brücke 38, welche zusätzliche Raum bietet, um so das Befestigungsloch 28 möglichst dicht an dem Rahmen 39 anzuordnen. Das kann insbesondere bei kleinen Größen (aber nicht beschränkt darauf) von Vorteil sein, um zwecks besseren Halts in der Spongiosa einen möglichst großen Abstand zu erreichen zu dem Verankerungskiel 8 und dem zu seiner Aufnahme erforderlichen Knochenhohlraum.

Wie man weiter aus Figur 1 gut erkennen kann, ist die Durchgangsöffnung 2 in mehrere Bereiche gegliedert. Den größten Bereich nimmt am Rand der Durchgangsöffnung typischerweise ein Aufnahmebereich 22 für den das Schaftstück 82 des Verankerungskiel 8 ein, an den sich ein lang gestreckter Bereich 23 der Durchgangsöffnung erstreckt, welcher typischerweise zur Aufnahme des flügelartigen Auslegers 83 ausgebildet ist. An dem fernen Ende hiervon ist typischerweise ein kreisrunder Aufnahmebereich 24 die für die Spannhülsen 84 am freien Ende des jeweiligen flügelartigen Auslegers 83 vorgesehen. Wie insbesondere durch einen Vergleich mit Figur 7 erkannt werden kann, ist die Durchgangsöffnung 2 mit ihren Bereichen 22, 23 und 24 auf den Verankerungskiel 8 bezüglich der Dimensionierung abgestimmt. Somit ergibt sich eine Aufnahme des Verankerungskiels 8 mit Spiel, das in Figur 7 durch die beiden gegenläufigen Pfeile visualisiert ist. Es ist so groß bemessen, dass ein ausreichender Freigang zu dem Verankerungskiel 8 mit seinen Auslegern 83 verbleibt und ausreichend Freiraum für eine Kompression der Schenkel 31, 32 und des Rahmens 39 verbleibt, ohne dass die Scheibensegmente 36 am Verankerungskiel 8 anschlagen. Es beträgt bei dem in Figur 7 dargestellten Beispiel etwa 1,5 bis 2 mm.

Die Skelettbauweise mit dem elastischen Rahmen 39 und den Scheibensegmenten 36 ermöglicht es, auf einfache Weise Varianten des Augments in größeren oder kleineren Größen bereitzustellen. Die Größe und/oder Anzahl der Scheibensegmente kann variiert werden. Indem beispielsweise eines der Scheibensegmente 36 weggelassen wird oder die Scheibensegmente 36, wie in Figur 5B) dargestellt ist, kleiner bemessen sind, kann eine kleinere Größe des Augments 1 gebildet werden. Oder es kann durch Hinzufügen eines Scheibensegments 36', wie in Figur 5A) dargestellt ist, oder dadurch, dass die Scheibensegmente 36 vergrößert werden, wie in Figur 8 dargestellt ist, eine größere Größe gebildet werden. Auf diese Weise ergeben sich Synergie-Effekte bei Bereitstellung von erfindungsgemäßen Augmenten in einem Augment-Satz mit verschiedenen Größen.

Die poröse Struktur 5 kann mit einer biokompatiblen Beschichtung 55 versehen sein, beispielsweise aus Kalziumphosphat zur weiteren Förderung von Knocheneinwuchs. Dies gilt für die poröse Struktur 5 aller Ausführungsformen.

Mehrere am Rahmen 39 angeordnete Biegescharniere 6 sind beispielhaft in Figur 8 dargestellt. Es versteht sich, dass die Biegescharniere bei den anderen dargestellten Augmenten 1 angeordnet sein können. Die Biegescharniere 6 sind außen an dem Rahmen 39 ausgebildet. Sie sind gebildet durch jeweils eine am Außenmantel 15 von der ersten zur zweiten Seite durchlaufende Nut 61, sodass in dem entsprechenden Bereich in Bezug auf die Materialstärke lediglich ein verhältnismäßig dünner durchgehender Streifen verbleibt. Damit ergibt sich eine lokale Materialschwächung. Die Biegescharniere 6 führen zu einer Verringerung der Biegesteifigkeit des Rahmens 39 insgesamt. Der Umfang ist einstellbar durch die Art und Anzahl der Biegescharniere 6.

Eine Detailansicht eines der Biegescharniere 6 gesehen aus der Blickrichtung, wie sie durch den mit der Markierung "IX" versehenen Pfeil dargestellt ist, ist in Figur 9A, B) gezeigt. Dargestellt ist jeweils ein Stück aus dem Außenmantel 15 mit einer Nut 61 in der Bildmitte. Es können optional Schlitzungen 62, wie in Figur 9A), oder Perforationen 63, wie in Figur 9B), vorgesehen sein. Die Schlitzungen 62 liegen vorzugsweise in einer Linie, um eine definierte Biegelinie zu erreichen; zwingend ist dies aber nicht. Die Perforationen sind clusterartig in zwei Gruppen 63' zusammengefasst. Zwischen den Schlitzungen 62 bzw. den Perforationen 63 verbleiben jeweils Materialbereiche, deren gemeinsame Breite bestimmend ist für die Biegesteifigkeit des jeweiligen Biegegelenks 6. Je weniger Breite verbleibt, desto weniger biegesteif ist das so gebildete Biegescharnier 6. Die runde Ausführung der Perforationen 63 bietet den Vorteil, dass sie eine besonders feinteilige Einstellung ermöglicht und ferner durch die Vermeidung von scharfen Ecken einen günstigen Verlauf der mechanischen Belastungslinien erreicht. Eine für die Langzeitstabilität schädliche Kerbwirkung kann auf diese Weise sicher vermieden werden.

Nachfolgend sei noch kurz die Explantation der aus einer Endoprothese, insbesondere Kniegelenkendoprothese, und dem scheibenförmigen Augment 1 erläutert. Es wird Bezug genommen auf Figur 4 und den dort dargestellten Sägeschlitz (siehe insbesondere den durch die gestrichelte Ellipse markierten Bereich), in den bei einer Explantation eine Säge eingeführt werden kann. Zur Explantation werden vorab die Befestigungsschrauben 98 herausgeschraubt und die Abstandseinsätze 96 ebenfalls entnommen. Nunmehr ist der Sägeschlitz von der Seite her bis zum Schaftstück 82 in der Mitte der Tibiaplatte 91 zugänglich. Die Unterseite der Tibiaplatte 91 kann somit mittels einer Säge (nicht dargestellt) freigeschnitten werden. Schließlich wird die zentrale Schraube 95 entfernt, und die Tibiaplatte 91 kann nunmehr abgenommen werden. Auf diese Weise wird das Augment 1 mit einer ggf. darauf angeordneten Zementschicht zugänglich, welche dann bei Bedarf ebenfalls entfernt werden kann. Gewünschtenfalls kann bei Bedarf das Augment 1 durch Zusammendrücken der Schenkel 31, 32 nach oben explantiert werden.

## Patentansprüche

1. Scheibenförmiges Augment zum Ausfüllen von Knochendefekten, insbesondere am Ende von langen Röhrenknochen wie Tibia, mit einer ersten Seite (11), einer zweiten Seite (12), einem Außenmantel (15) an Lateralseiten sowie einer Innenwandung für eine von der ersten zur zweiten Seite laufende Durchgangsöffnung (2) für einen Verankerungskiel (8) einer an der zweiten Seite angeordneten Endoprothese (9), wobei das Augment eine generell C-förmige Gestalt mit zwei die Durchgangsöffnung flankierenden Schenkeln (31, 32) aufweist,
**dadurch gekennzeichnet, dass**
ein Verbindungsstück (30) zwischen den Schenkeln (31, 32) gelenkartig ausgeführt ist und mit den Schenkeln (31, 32) elastisch zusammenwirkt, so dass ein Rahmen (39) gebildet ist, und bei Kompression der Schenkel (31, 32) eine nach außen gerichtete Rückstellkraft erzeugt ist, wobei die Schenkel ausgeführt sind in Skelettbauweise mit mehreren durch Schlitze (34) getrennten, nebeneinanderliegenden Scheibensegmenten (36), die über Stege (37) an dem Rahmen angeordnet sind.

2. Scheibenförmiges Augment nach Anspruch 1, **dadurch gekennzeichnet, dass** die erste Seite (11) als poröse Struktur (5) ausgeführt ist, wobei vorzugsweise Kanten (50) massiv ausgeführt sind, und/oder der Außenmantel (15) als eine Knocheneinwuchs fördernde poröse Struktur (5) ausgeführt ist.

3. Scheibenförmiges Augment nach Anspruch 2, **dadurch gekennzeichnet, dass** die poröse Struktur (5) miteinander in der Tiefe des Materials verbundenen Poren umfasst, die vorzugsweise eine Weite von 0,4 bis 1,0 mm aufweisen,
und/oder
die poröse Struktur (5) poröse Bereiche aufweist, die von einem massiven Rand umrahmt sind.

4. Scheibenförmiges Augment nach einem der Ansprüche 1 bis 3, **dadurch gekennzeichnet, dass** die Schenkel (31, 32) einen massiven Kern (33) aufweisen, an dessen Außenseite Taschen (35) gebildet sind, in denen poröse Struktur (5) angeordnet ist.

5. Scheibenförmiges Augment nach einem der Ansprüche 1 bis 4, **dadurch gekennzeichnet, dass** die Schenkel (31, 32) insbesondere mit ihren Scheibensegmenten (36) aus einer porösen Struktur (5) gebildet sind.

6. Scheibenförmiges Augment nach einem der vorangehenden Ansprüche, **dadurch gekennzeichnet, dass** Innenwandungen an den Schenkeln (31, 32) und/oder den Scheibensegmenten (36) massiv ausgeführt ist.

7. Scheibenförmiges Augment nach einem der vorangehenden Ansprüche, **dadurch gekennzeichnet, dass** die Schlitze (34) so eng bemessen sind, dass sie als Spaltdichtung für Knochenzement fungieren, vorzugsweise maximal 0,7 mm weit sind, weiter vorzugsweise maximal 0,4 mm.

8. Scheibenförmiges Augment nach einem der vorangehenden Ansprüche, **dadurch gekennzeichnet, dass** der Rahmen (39) als ein die Gesamtheit der Scheibensegmente (36) mindestens hälftig umschließender Außenrand ausgeführt ist, an dessen Außenseite der Außenmantel (15) angeordnet ist.

9. Scheibenförmiges Augment nach einem der vorangehenden Ansprüche, **dadurch gekennzeichnet, dass** in dem Rahmen (39) und/oder den Scheibensegmenten (36) mindestens ein Befestigungsloch (28) zur Aufnahme einer Befestigungsschraube (29) vorgesehen ist, wobei das Befestigungsloch (27) vorzugsweise einen massiven Lochmantel (27) aufweist,
wobei insbesondere das Befestigungsloch (28) in einem der Scheibensegmente (36) angeordnet ist, und weiter vorzugsweise dieses Scheibensegment (36) mittels einer bezüglich der Stege (37) breiteren Brücke (38) am Rahmen angeordnet ist.

10. Scheibenförmiges Augment nach einem der vorangehenden Ansprüche, **dadurch gekennzeichnet, dass** die Durchgangsöffnung (2) ein langgestreckter Aufnahmeraum für einen Verankerungskiel (8) ist, und/oder die Durchgangsöffnung (2) zu einer Seite hin offen ist, und/oder die Durchgangsöffnung weniger als die Hälfte, vorzugsweise weniger als ein Drittel des Volumens des Augments einnimmt.

11. Scheibenförmiges Augment nach einem der vorangehenden Ansprüche, **dadurch gekennzeichnet, dass** der Außenmantel (15) konisch geneigt ist, vorzugsweise mit einem Winkel von 5 bis 10° zur ersten Seite (11) hin verjüngend, und/oder an dem Außenmantel (15) radial nach außen gerichtete Spikes (16) vorgesehen sind, und/oder
die poröse Struktur (5) mit einer knocheneinwuchsfördernden Beschichtung (55), insbesondere umfassend Kalziumphosphat, versehen ist, und/oder an nicht-porösen Bereichen mit einer bioziden Beschichtung versehen ist.

12. Scheibenförmiges Augment nach einem der vorangehenden Ansprüche, **dadurch gekennzeichnet, dass** an dem Rahmen (39) mindestens ein Biegescharnier (6) vorgesehen ist, das dazu ausgebildet ist, eine zusätzliche Kompression der Schenkel (31, 32) zu ermöglichen und eine nach außen gerichtete Rückstellkraft zu erzeugen, wobei
vorzugsweise das mindestens eine Biegescharnier (6) gebildet ist durch eine lokale Materialschwächung, insbesondere in Form einer Nut (61), einer Schlitzung (62) und/der Perforationen (63).

13. Scheibenförmiges Augment nach Anspruch 12, **dadurch gekennzeichnet, dass** bei mehreren Biegescharnieren (6) diese sich bezüglich ihrer Materialschwächung unterscheiden, so dass sie bei Kompression verschieden hohe Rückstellkräfte aufweisen.

14. Anordnung aus einer Endoprothese, insbesondere einer Kniegelenkendoprothese, und dem scheibenförmigen Augment (1) nach einem der vorangehenden Ansprüche, **dadurch gekennzeichnet, dass** ein Verankerungskiel (8) der Endoprothese (9) in die Durchgangsöffnung (2) aufgenommen ist.

15. Anordnung nach Anspruch 14, **dadurch gekennzeichnet, dass** das scheibenförmige Augment (1) außenkonturähnlich aber untermaßig bezogen auf die Endoprothese, insbesondere Tibiaplatte (91), ist.

16. Anordnung nach Anspruch 14 oder 15, **dadurch gekennzeichnet, dass** das scheibenförmige Augment (1) zur Unterstützung einer linken oder rechten Hälfte der Endoprothese (9) halbseitig ausgeführt ist, und/oder
die Durchgangsöffnung (2) zur Aufnahme von dem Verankerungskiel (8) und dessen flügelartigen Auslegern (83) mit einem definierten Freiraum von vorzugsweise 1 bis 3 mm bemessen ist.

## Claims

1. A disc-shaped augment for filling bone defects, in particular at the end of tubular bones such as the tibia, with a proximal side (12), a distal side (11), an outer sheath (15) on lateral sides and an inner wall for a through-opening (2), running from the proximal side to the distal side, for an anchoring keel (8) of an endoprosthesis (9) arranged on the proximal side, wherein the augment has a generally C-shaped form with two legs (31, 32) flanking the through-opening,
**characterized in that**
a connecting piece (30) between the legs (31, 32) is designed in the manner of a joint and interacts elastically with the legs (31, 32) so that a frame (39) is formed and an outwardly directed restoring force is generated upon compression of the legs (31, 32), wherein the legs are designed in a skeleton construction with a plurality of adjacent disc segments (36) separated by slots (34) and arranged on the frame via webs (37).

2. The disc-shaped augment according to claim 1, **characterized in that** the first side (11) is designed as a porous structure (5), wherein edges (50) are preferably designed to be solid and/or the outer sheath (15) is designed as a porous structure (5) promoting bone ingrowth.

3. The disc-shaped augment according to claim 2, **characterized in that** the porous structure (5) comprises pores connected to one another in the depth of the material, which preferably have a width of 0.4 to 1.0 mm,
and/or
the porous structure (5) has porous regions which are framed by a solid edge.

4. The disc-shaped augment according to any one of claims 1 to 3, **characterized in that** the legs (31, 32) have a solid core (33) on the outside of which pockets (35) are formed, in which the porous structure (5) is arranged.

5. The disc-shaped augment according to any one of claims 1 to 4, **characterized in that** the legs (31, 32) are formed from a porous structure (5), in particular with their disc segments (36).

6. The disc-shaped augment according to any one of the preceding claims, **characterized in that** the inner walls on the legs (31, 32) and/or the disc segments (36) are solid.

7. The disc-shaped augment according to any one of the preceding claims, **characterized in that** the slots (34) are dimensioned so narrowly that they act as a gap seal for bone cement, having preferably a maximum width of 0.7 mm, more preferably a maximum width of 0.4 mm.

8. The disc-shaped augment according to any one of the preceding claims, **characterized in that** the frame (39) is designed as an outer edge which surrounds at least half of the entirety of the disc segments (36), on the outside of which the outer sheath (15) is arranged.

9. The disc-shaped augment according to any one of the preceding claims, **characterized in that** at least one fastening hole (28) for receiving a fastening screw (29) is provided in the frame (39) and/or in the disc segments (36), wherein the fastening hole (27) preferably has a solid perforated sheath (27),
wherein in particular the fastening hole (28) is arranged in one of the disc segments (36), and further preferably this disc segment (36) is arranged on the frame by means of a bridge (38) which is wider with respect to the webs (37).

10. The disc-shaped augment according to any one of the preceding claims, **characterized in that** the through-opening (2) is an elongate receiving space for an anchoring keel (8) and/or the through-opening (2) is open on one side and/or the through-opening occupies less than half, preferably less than a third, of the volume of the augment.

11. The disc-shaped augment according to any one of the preceding claims, **characterized in that** the outer sheath (15) is conically inclined, preferably tapering to the first side (11) at an angle of 5 to 10°, and/or
radially outwardly directed spikes (16) are provided on the outer sheath (15), and/or
the porous structure (5) is provided with a bone ingrowth-promoting coating (55), in particular comprising calcium phosphate, and/or is provided with a biocidal coating on nonporous regions.

12. The disc-shaped augment according to any one of the preceding claims, **characterized in that** at least one bending hinge (6) is provided on the frame (39), which hinge is designed to enable additional compression of the legs (31, 32) and to generate an outwardly directed restoring force, wherein
preferably the at least one bending hinge (6) is formed by a local material weakening, in particular in the form of a groove (61), a slit (62) and/or perforations (63).

13. The disc-shaped augment according to claim 12, **characterized in that** if there are several bending hinges (6), these differ in terms of their material weakening, so that they have different restoring forces when compressed.

14. An arrangement of an endoprosthesis, in particular of a knee joint endoprosthesis, and the disc-shaped augment (1) according to any one of the preceding claims, **characterized in that** an anchoring keel (8) of the endoprosthesis (9) is accommodated in the through-opening (2).

15. The arrangement according to claim 14, **characterized in that** the disc-shaped augment (1) is similar in outer contour but undersized in relation to the endoprosthesis, in particular the tibial plate (91).

16. The arrangement according to claim 14 or 15, **characterized in that** the disc-shaped augment (1) is designed to support a left or right half of the endoprosthesis (9) on one half of the side, and/or
**in that** the through-opening (2) for receiving the anchoring keel (8) and its wing-like extensions (83) is dimensioned with a defined free space of preferably 1 to 3 mm.

## Revendications

1. Insert en forme de disque pour combler les défauts osseux, en particulier à l'extrémité des os longs tubulaires tels que le tibia, avec un premier côté (11), un second côté (12), une enveloppe extérieure (15) sur les côtés latéraux et une paroi intérieure pour une ouverture traversante (2) évoluant du premier côté au second côté pour une quille d'ancrage (8) d'une endoprothèse (9) agencée sur le second côté, dans lequel l'insert présente une forme générale en C avec deux pattes (31, 32) encadrant l'ouverture traversante,
**caractérisé en ce que**
une pièce de liaison (30) entre les pattes (31, 32) est conçue à la manière d'une articulation et interagit élastiquement avec les pattes (31, 32) de sorte qu'un cadre (39) est formé et qu'une force de rappel dirigée vers l'extérieur est générée lors de la compression des pattes (31, 32), dans lequel les pattes sont conçues dans une construction squelette avec une pluralité de segments de disque adjacents (36) séparés par des fentes (34), lesquels segments sont disposés sur le cadre par l'intermédiaire d'entretoises (37).

2. Insert en forme de disque selon la revendication 1, **caractérisé en ce que** la première face (11) est conçue comme une structure poreuse (5), dans lequel les bords (50) sont de préférence conçus de manière massive, et/ou l'enveloppe extérieure (15) est conçue comme une structure poreuse (5) favorisant la croissance osseuse.

3. Insert en forme de disque selon la revendication 2, **caractérisé en ce que** la structure poreuse (5) présente des pores reliés les uns aux autres dans la profondeur du matériau, qui présentent de préférence une largeur de 0,4 à 1,0 mm,
et/ou
la structure poreuse (5) présente des zones poreuses qui sont encadrées par un bord solide.

4. Insert en forme de disque selon l'une des revendications 1 à 3, **caractérisé en ce que** les pattes (31, 32) présentent un noyau massif (33) à l'extérieur duquel sont formées des poches (35) dans lesquelles la structure poreuse (5) est disposée.

5. Insert en forme de disque selon l'une des revendications 1 à 4, **caractérisé en ce que** les pattes (31, 32), en particulier avec leurs segments de disque (36), sont formées d'une structure poreuse (5).

6. Insert en forme de disque selon l'une des revendications précédentes, **caractérisé en ce que** les parois intérieures sont conçues de manière massive au niveau des pattes (31, 32) et/ou des segments de disque (36).

7. Insert en forme de disque selon l'une des revendications précédentes, **caractérisé en ce que** les fentes (34) sont dimensionnées si étroitement qu'elles agissent comme un joint d'étanchéité pour le ciment osseux, de préférence d'un maximum de 0,7 mm de large, de manière davantage préférée d'un maximum de 0,4 mm.

8. Insert en forme de disque selon l'une des revendications précédentes, **caractérisé en ce que** le cadre (39) est réalisé sous la forme d'un bord extérieur qui entoure au moins la moitié de la totalité des segments de disque (36), à l'extérieur desquels est disposée l'enveloppe extérieure (15).

9. Insert en forme de disque selon l'une des revendications précédentes, **caractérisé en ce qu'**au moins un trou de fixation (28) destiné à recevoir une vis de fixation (29) est prévu dans le cadre (39) et/ou les segments de disque (36), dans lequel le trou de fixation (27) présente de préférence un boîtier perforé solide (27),
dans lequel en particulier le trou de fixation (28) est disposé dans l'un des segments de disque (36), et de préférence encore ce segment de disque (36) est disposé sur le cadre au moyen d'un pont (38) plus large par rapport aux âmes (37).

10. Insert en forme de disque selon l'une des revendications précédentes, **caractérisé en ce que** l'ouverture traversante (2) est un espace allongé de réception pour une quille d'ancrage (8), et/ou que l'ouverture traversante (2) est ouverte sur un côté, et/ou que l'ouverture traversante occupe moins de la moitié, de préférence moins du tiers, du volume de l'insert.

11. Insert en forme de disque selon l'une des revendications précédentes, **caractérisé en ce que** l'enveloppe extérieure (15) est inclinée coniquement, de préférence effilé selon un angle de 5 à 10° vers le premier côté (11), et/ou
des pointes dirigées radialement vers l'extérieur (16) sont prévues sur le boîtier extérieur (15), et/ou
la structure poreuse (5) est munie d'un revêtement favorisant la croissance osseuse (55), comprenant en particulier du phosphate de calcium, et/ou est munie, sur les zones non poreuses, d'un revêtement biocide.

12. Insert en forme de disque selon l'une des revendications précédentes, **caractérisé en ce qu'**au moins une charnière de flexion (6) est prévue sur le cadre (39), laquelle est conçue pour permettre une compression supplémentaire des pattes (31, 32) et pour générer une force de rappel dirigée vers l'extérieur, dans lequel
de préférence, l'au moins une charnière de flexion (6) est formée par un affaiblissement local du matériau, notamment sous la forme d'une rainure (61), d'une fente (62) et/ou de perforations (63).

13. Insert en forme de disque selon la revendication 12, **caractérisé en ce que**, s'il y a plusieurs charnières de flexion (6), celles-ci diffèrent en termes d'affaiblissement de matériau, de sorte qu'elles présentent des forces de rappel différentes lorsqu'elles sont comprimées.

14. Disposition d'une endoprothèse, en particulier d'une endoprothèse d'articulation de genou, et de l'insert en forme de disque (1) selon l'une des revendications précédentes, **caractérisée en ce qu'**une quille d'ancrage (8) de l'endoprothèse (9) est reçue dans l'ouverture traversante (2).

15. Disposition selon la revendication 14, **caractérisée en ce que** l'insert en forme de disque (1) est de contour extérieur similaire, mais sous-dimensionné par rapport à l'endoprothèse, en particulier le plateau tibial (91).

16. Disposition selon la revendication 14 ou 15, **caractérisée en ce que** l'insert en forme de disque (1) est conçu pour supporter une moitié gauche ou droite de l'endoprothèse (9) d'un côté, et/ou
l'ouverture traversante (2) pour la réception de la quille d'ancrage (8) et de ses prolongements en forme d'ailes (83) est dimensionnée à raison d'un espace libre défini de préférence de 1 à 3 mm.
